Europäisches Patentamt
European Patent Office
Office européen des brevets

(19)

(11) Publication number: **0 079 141 A1**

(12) **EUROPEAN PATENT APPLICATION**

(21) Application number: 82305512.4

(22) Date of filing: 18.10.82

(51) Int. Cl.³: C 07 C 97/10
C 07 C 93/14, A 61 K 31/135

(30) Priority: 20.10.81 JP 166453/81

(43) Date of publication of application: 18.05.83 Bulletin 83/20

(84) Designated Contracting States: AT BE CH DE FR GB IT LI LU NL SE

(71) Applicant: ONO PHARMACEUTICAL CO., LTD.
No. 14, Doshomachi 2-chome Higashi-ku
Osaka-shi Osaka(JP)

(72) Inventor: Miyamoto, Tsumoru
1-40 Koaza Moriyama Aza Tono
Johyoh-shi Kyoto(JP)

(72) Inventor: Watsuka, Hirohisa
3-3-708 Miyano-cho
Takatsuki-shi Osaka(JP)

(72) Inventor: Hashimoto, Shinsuke
2-6-905, Shirakawa 3-chome
Ibaraki-shi Osaka(JP)

(72) Inventor: Itoh, Hiroyuki
16-20 Yamate-dai 3-chome
Ibaraki-shi Osaka(JP)

(72) Inventor: Mohri, Tetsuya
14-3 Ohsumigaoka 4-chome, Tanabe-cho
Tsuzuki-gun Kyoto(JP)

(72) Inventor: Hayashi, Masaki
5-10 Nanpei-dai 4-chome
Takatsuki-shi Osaka(JP)

(74) Representative: Pearce, Anthony Richmond et al,
Marks & Clerk Alpha Tower Suffolk Street Queensway
Birmingham B1 1TT(GB)

(54) **Aminoresorcinol derivatives.**

(57) AMINORESORCINOL derivatives of the general formula:

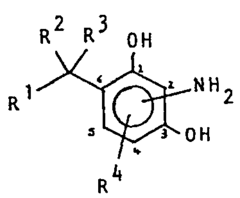

(I)

[wherein $R^1$ represents an alkyl group containing from 1 to 6 carbon atoms, or a group of the general formula:

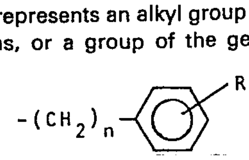

(II)

(in which $R^5$ represents a hydrogen atom, a halogen atom, an alkyl group containing from 1 to 4 carbon atoms, an alkoxy group containing from 1 to 4 carbon atoms, a carboxy group, an amino group, a carbamoyl group, a sulfamoyl group or a cyano group and n represents zero or an integer of 1 or 2), one of $R^2$ and $R^3$ represents a hydrogen atom and the other represents a hydroxy group or an alkoxy group containing from 1 to 4 carbon atoms or $R^2$ and $R^3$ together represent an oxo group, and $R^4$ represents a hydrogen atom, a halogen atom, an alkyl group containing from 1 to 6 carbon atoms or a phenylthio group, and the amino group and the group $R^4$ in the general formula (I) can be attached to the position 2 and 4, or 4 and 2 of the resorcinol ring, respectively], and pharmaceutically-acceptable acid addition salts thereof, possess a strong inhibitory effect on 5-lipoxygenase specifically, or 5-lipoxygenase and cyclooxygenase at the same time, and therefore, are useful as treating and preventing agents for diseases which induced by leukotrienes, e.g. allergic tracheal and bronchial diseases or allergic lung diseases, allergic shocks or various allergic inflammations, and various inflammations induced by prostaglandins.

EP 0 079 141 A1

Croydon Printing Company Ltd.

## SPECIFICATION

## AMINORESORCINOL DERIVATIVES

This invention is concerned to new aminoresorcinol derivatives which have an inhibitory activity on 5-lipoxygenase specifically or an inhibitory activity on 5-lipoxygenase and cyclooxygenase at the same time, the process for their preparation and pharmaceutical compositions containing them as an active ingredient.

Up to now, several compounds which inhibit lipoxygenase have been known, that is to say, 5,8,11,14-eicosatetraynoic acid (i.e. EYTA), 5,8,11-eicosatriynoic acid, acetone phenylhydrazone (i.e. APH), phenidone (i.e. 1-phenyl-3-pyrazolidone), NDGA (nordihydroguaiaretic acid), BW-775C (3-amino-1-p-trifluoromethyl-pyrazole), 3-amino-1-p-chlorophenyl-2-pyrazoline, benoxaprofene.

These above compounds inhibit all lipoxygenases and other enzymes in the arachidonate cascade at the same time, or inhibit all lipoxygenases of 5-, 12- and 15-lipoxygenase [see Gendai Iryo , 12, 1065 (1980)].

The compounds of the present invention are particularly superior in having selective inhibitory activity on 5-lipoxygenase of many lipoxygenases,which react at the first stage of producing various kinds of leukotrienes from arachidonic acid in a living body and further not inhibiting other enzymes in the arachidonate cascade, or in having inhibitory activity on 5-lipoxygenase and cyclooxygenase which reacts at the first step of producing prostaglandins from arachidonic acid similarly, at the same time.

This invention is concerned to new aminoresorcinol derivatives which have an inhibitory activity on 5-lipoxygenase, or an inhibitory activity on 5-lipoxygenase and on cyclooxygenase at the same time, the process for their preparation and pharmaceutical compositions containing them as an active ingredient. More particularly, there are a group of compounds called leukotrienes, which are biosynthesized from arachidonic acid by a series of reactions in a living body and 5-lipoxygenase is related to the first step of this reaction. Similarly, prostaglandins are biosynthesized from arachidonic acid by another series of reactions, and cyclooxygenase is related to the first step of such reaction. This invention is concerned to new aminoresorcinol derivatives which strongly inhibit 5-lipoxygenase or strongly inhibit 5-lipoxygenase and cyclooxygenase at the same time, and which are, therefore, useful as treating and preventing agentsfor diseases which induced by leukotrienes, e.g. allergic tracheal and bronchial diseases or allergic lung diseases, allergic shocks or various allergic inflammations, and various inflammations induced by prostaglandins, and further, this invention is concerned to the process for their preparation and pharmaceutical compositions comprising them as an active ingredient.

In the study of prostaglandins (abbreviated as PG hereafter), many important discoveries have been made continuously in recent years. And so it was found a large change in the direction of the research and development of PG. In the compounds which have been newly found or newly confirmed their structure in PG family, it can be said that PG endoperoxides, (i.e. $PGG_2$ and $PGH_2$), thromboxane $A_2$ (abbreviated as $TXA_2$ hereafter), prostacyclin (i.e. $PGI_2$) and leukotriene C, D and E

(abbreviated as LTC, LTD and LTE, respectively, hereafter) etc. have especially strong and unique biological activities.

All the compounds of PG family containing various PG previously known well in addition to the above compounds, are biosynthesized from the same mother compound, i.e. arachidonic acid in a living body and, therefore, the metabolic routes starting from arachidonic acid is called "Arachidonate cascade" as a whole. The detailed explanation of each route and the pharmacological character of each metabolite are described in Igaku no Ayumi, 114, 378 (1980), ibid, 114, 462 (1980), ibid, 114, 866 (1980), ibid, 114, 929 (1980), Gendai Iryo, 12, 909 (1980), ibid, 12, 1029 (1980), ibid, 12, 1065 (1980) and ibid, 12, 1105 (1980) etc.

The arachidonate cascade can be largely divided into two routes; one is the route that cyclooxygenase acts on arachidonic acid to convert, via $PGG_2$ and further $PGH_2$, into various PG, e.g. prostaglandin $F_2\alpha$ (abbreviated $PGF_2\alpha$ hereafter), prostaglandin $E_2$ (abbreviated $PGE_2$ hereafter), $PGI_2$, $TXA_2$, and the other is the route that lipoxygenase acts on arachidonic acid to convert, via hydroperoxyeicosatetraenoic acid (abbreviated HPETE hereafter), into hydroxyeicosatetraenoic acid (abbreviated as HETE hereafter) or leukotrienes.

As the former route is well known, it is not described in the present specification in detail. See Prostaglandin (1978), edited by Makoto Katori, published by Kohdan-sha.

Concerning the latter route, it has been known that various compounds are produced according to the following Scheme A.

SCHEME A

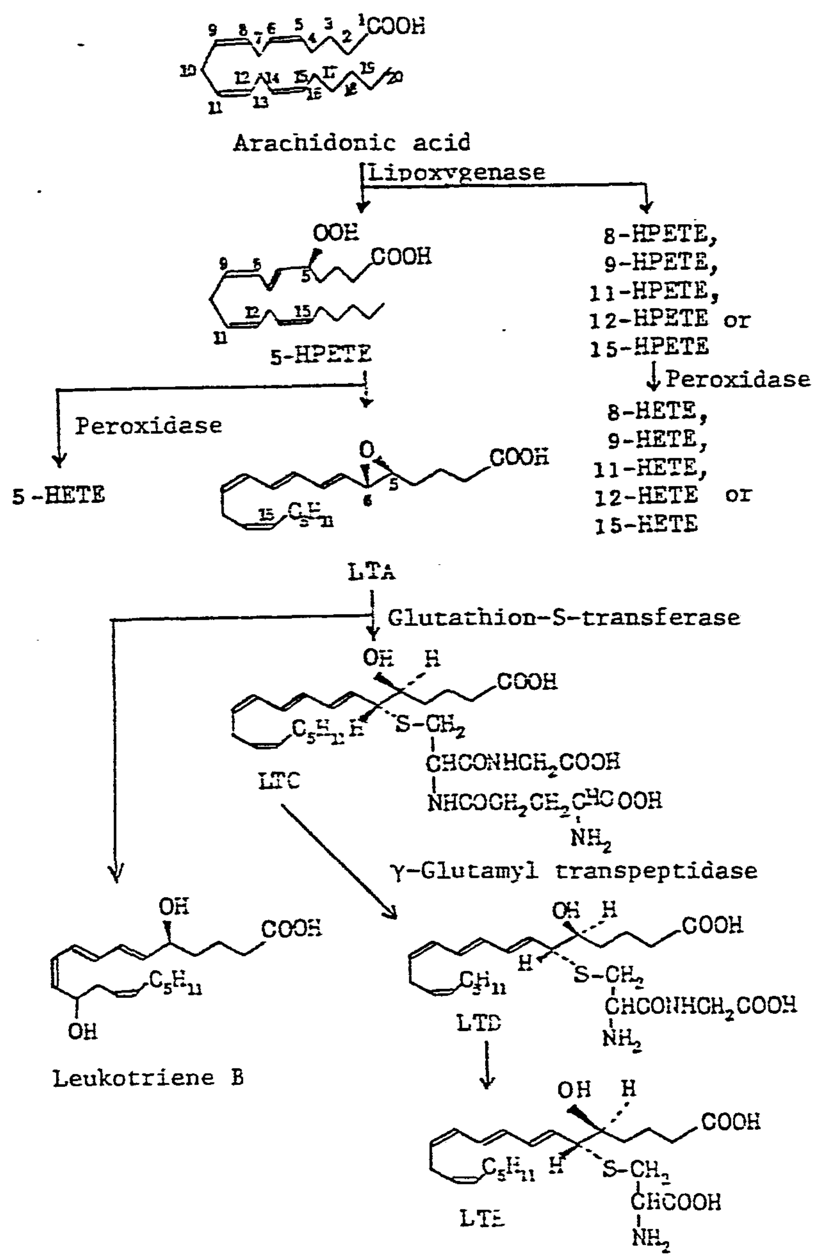
Arachidonic acid
Lipoxygenase
5-HPETE
8-HPETE,
9-HPETE,
11-HPETE,
12-HPETE or
15-HPETE
Peroxidase
8-HETE,
9-HETE,
11-HETE,
12-HETE or
15-HETE
Peroxidase
5-HETE
LTA
Glutathion-S-transferase
LTC
γ-Glutamyl transpeptidase
Leukotriene B
LTD
LTE

Besides being metabolized through a well known route, i.e. the route via PG endoperoxides, arachidonic acid is also metabolized through another route by the action of lipoxygenase. That is to say, arachidonic acid is metabolized by the action of lipoxygenase, e.g. 5-lipoxygenase, 12-lipoxygenase and 15-lipoxygenase, to 5-HPETE, 12-HPETE and 15-HPETE, respectively, having following formulae:

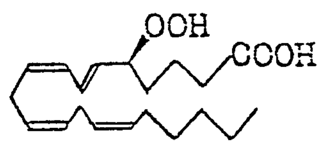

,

5-HPETE

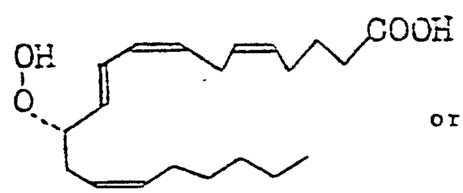

or

12-HPETE

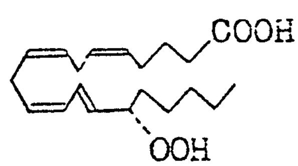

15-HPETE

These HPETE are converted into 5-HETE, 12-HETE and 15-HETE, respectively, by the action of peroxidase converting a hydroperoxy group into a hydroxy group. Furthermore, LTA is also produced from 5-HPETE. LTA is converted into leukotriene B (abbreviated as LTB hereafter) enzymatically or not enzymatically, or is converted into LTC by the action of glutathion-S-transferase. Further, LTC is converted into LTD by the action of γ-glutamyl transpeptidase.

Moreover, it was recently defined that LTD is metabolized to LTE [see Biochem. Biophys. Res. Commun., 91, 1266 (1979) and Prostaglandins, 19(5), 645 (1980)].

It was found that LTC and LTD are identical with SRS (slow reacting substance) or SRS-A (slow reacting substance of anaphylaxis) which was well known before [see Proc. Natl. Acad. Sci. USA, 76, 4275 (1979), Biochem. Biophys. Res. Commun., 91, 1266 (1979), Proc. Natl. Acad. Sci. USA, 77, 2014 (1980) and Nature, 285, 104 (1980)]. So it can be understood that the pharmacological characters of these leukotrienes are the same as those of SRS or SRS-A.

Feldberg et al. reported that a substance is released when perfusing cobra venom through isolated lung or incubating cobra venom with vitellus. The substance was named SRS and it has been found that SRS constricts ilem isolated from guinea pig slowly and continually [see J. Physiol., 94, 187 (1938)].

Moreover, Kellaway et al. showed the relation between SRS-A and allergic reaction from the fact that SRS-A is released when an antigen is sensitized to perfusing lung isolated from guinea pig [see Quant. J. Exp. Physiol., 30, 121 (1940)].

Brocklehurst reported that when the antigen is sensitized to a

lung fragment isolated from a bronchial asthmatics whose specific antigen is defined, by an operation, histamine and SRS-A are released and strongly constrict bronchial muscle. Since such constriction can not be prevented by an antihistaminic agent, he suggested that SRS-A is an important bronchoconstrictor in an asthmatic paroxysm [see Progr. Allergy, 6, 539 (1962)].

Since then, many reports were published, for instance, SRS-A prepared from human lung slice constrict a tracheal spiral of normal human [see Int. Arch. Allergy Appl. Immunol., 38, 217 (1970)]; when SRS-A prepared from rats is injected intravenously to guinea pig, significant increase of purlmonory resistance is observed [see J. Clin. Invest., 53, 1679 (1974)]; in addition, a subcutaneous injection of SRS-A to guinea pig, rat and monkey makes vascular permeability to enhance. [see Advances in Immunology, 10, 105 (1969), J. Allergy Clin. Immunol., 621, 371 (1978), Prostaglandins, 19(5), 779 (1980) etc.].

Generally, the substance released by immunological reaction is called SRS-A. On the other hand, the substance released by non-immunological reaction such as calcium ionophore is called SRS. However, the above two substances have many similarities each other and, therefore, it is considered they would probably be the same substance.

Based on result of these studies, various leukotrienes (LTC, LTD and LTE, and further other leukotrienes which may be confirmed their structures in future) biosynthesized from arachidonic acid via LTA, are considered to be important factors relating to the appearance of allergic tracheal and bronchial disease, allergic lung disease, allergic shock and various allergic inflammations.

Recently, it was proposed that PG is an important chemical

mediator of inflammatory reaction. For example, it was reported that PGE enhances a vascular permeability and a pain, and has a vasodilative action, pyrogenetic action and chemotactic action [see Prostaglandin (1978), edited by Makoto Katori, published by Kohdan-sha]. Furthermore, it was reported that $PGI_2$ alone does not affected vascular permeability, but it enhances vascular permeability of histamine [see Prostaglandins, 15, 557 (1978)]. Moreover, the most recent report indicated that in order to suppress such action of PG, it is more effective to inhibit not only 5-lipoxygenase but also cyclooxygenase at the same time [see Biochemical Pharmacology, 28, 1959 (1979) and European Journal of Pharmacology, 66, 81 (1980)].

As the result of energetic investigations in order to find new compounds inhibiting 5-lipoxygenase, or inhibiting 5-lipoxygenase and cyclooxygenase at the same time in a living body, and so being effective for prevention and treatment of not only various allergic diseases induced by leukotrienes but also inflammations induced by PGs, we have found the compounds which can be achieved that purpose, having completed this invention.

The present invention accordingly provides new aminoresorcinol derivatives of the general formula:

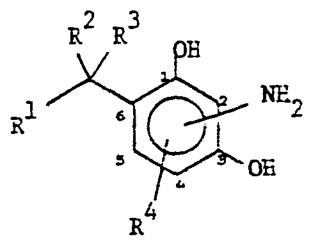

(I)

[wherein $R^1$ represents an alkyl group containing from 1 to 6 carbon atoms, or a group of the general formula:

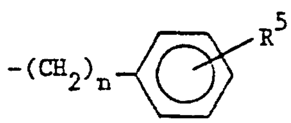

(II)

(in which $R^5$ represents a hydrogen atom, a halogen atom, an alkyl group containing from 1 to 4 carbon atoms, an alkoxy group containing from 1 to 4 carbon atoms, a carboxy group, an amino group, a carbamoyl group, a sulfamoyl group or a cyano group and n represents zero or an integer of 1 or 2), one of $R^2$ and $R^3$ represents a hydrogen atom and the other represents a hydroxy group or an alkoxy group containing from 1 to 4 carbon atoms or $R^2$ and $R^3$ together represent an oxo group, and $R^4$ represents a hydrogen atom, a halogen atom, an alkyl group containing from 1 to 6 carbon atoms or a phenylthio group, and the amino group and the group $R^4$ in the general formula (I) can be attached to the position 2 and 4, or 4 and 2 of the resorcinol ring, respectively],
and pharmaceutically-acceptable acid addition salts thereof.

As will be apparent to those skilled in the art, when one of $R^2$ and $R^3$ represents a hydroxy or alkoxy group, the carbon atom which $R^2$ and $R^3$ are attached to, will become center of chirality. Further

centers of chirality may occur when the alkyl groups represented by $R^1$, $R^4$ or $R^5$, or the alkyl moiety of the alkoxy groups represented by one of $R^2$ and $R^3$, or $R^5$, are branched-chain. The presence of chirality leads, as is well known, to the existence of isomerism. However, all isomers of general formula (I) and mixtures thereof are to be considered within the the scope of general formula (I).

In this specification, it is to be understood that the alkyl group and the alkyl moiety of the alkoxy groups may be straight- or branched-chain.

Examples of the alkyl group containing from 1 to 6 carbon atoms represented by $R^1$ and $R^4$, are methyl, ethyl, propyl, butyl, pentyl and hexyl and their isomers.

Examples of the alkyl group containing from 1 to 4 carbon atoms represented by $R^5$, are methyl, ethyl, propyl and butyl and their isomers.

Examples of the alkoxy group containing from 1 to 4 carbon atoms represented by one of $R^2$ and $R^3$, and $R^5$, are methoxy, ethoxy, propoxy and butoxy and their isomers.

Examples of the halogen atom represented by $R^4$ and $R^5$, are fluorine, chlorine, bromine and iodine atom.

Examples of the group represented by the formula $-(CH_2)_n-$, are single bond, methylene and ethylene.

Preferred examples of $R^1$ are the pentyl and hexyl groups and the group of the general formula (II) in which $R^5$ and n are hereinbefore defined. More preferred examples of $R^1$ are the group of the general formula (II) in which $R^5$ is a hydrogen or halogen atom, or an alkyl or alkoxy group containing from 1 to 4 carbon atoms. Most preferred

examples of $R^1$ are the group of the general formula (II) in which $R^5$ is a hydrogen, chlorine or fluorine atom. Preferably $R^2$ and $R^3$ together represents an oxo group, and further, one of $R^2$ and $R^3$ represents a hydrogen atom and the other represents a hydroxy, methoxy or ethoxy group. Preferred examples of $R^4$ are a hydrogen and halogen atoms and a tert-butyl group, and more preferred examples of $R^4$ are a hydrogen, chlorine and fluorine atoms. Preferably the amino group in the general formula (I ) is attached to the position 2 of the resorcinol ring and the group $R^4$ is attached to the position 4 of the said ring.

According to the present invention, aminoresorcinol derivatives of the general formula (I), wherein one of $R^2$ and $R^3$ represents a hydrogen atom and the other represents an alkoxy group containing from 1 to 4 carbon atoms, i.e. compounds of the general formula:

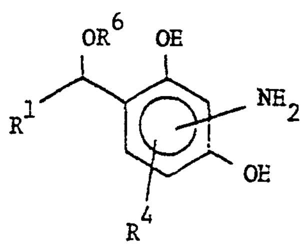

(IA)

(wherein $R^6$ represents an alkyl group containing from 1 to 4 carbon atoms and the other symbols are as hereinbefore defined) may be prepared from aminoresorcinol derivatives of the general formula (I), wherein one of $R^2$ and $R^3$ represents a hydrogen atom and the other represents a hydroxy group, i.e. compounds of the general formula:

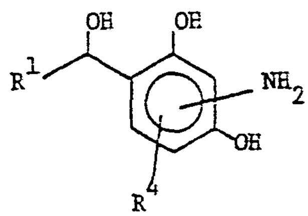

(IB)

(wherein the various symbols are as hereinbefore defined) by etherification of the secondary alcohol to an alkoxy group.

The converting method is well known, for example, by using an acid such as hydrochloric acid, nitric acid, p-toluenesulfonic acid etc. as a catalyst, in corresponding alkanol such as methanol, ethanol, propanol, butanol etc. at a temperature from 0°C to the refluxing temperature of the alkanol.

Aminoresorcinol derivatives of the general formula (IB) may be prepared from aminoresorcinol derivatives of the general formula (I), wherein $R^2$ and $R^3$ together represents an oxo group, i.e. compounds of the general formula:

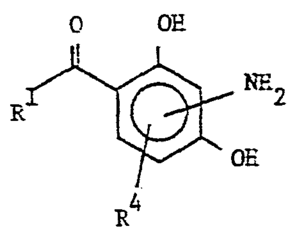

(IC)

(wherein the various symbols are as hereinbefore defined) by reduction to convert the oxo group to a hydroxy group.

The reduction may be carried out, for example, in an atmosphere of hydrogen using a catalyst, e.g. palladium-carbon, palladium black in an organic solvent at a temperature from 0°C to 40°C, or using metal hydride, e.g. lithium aluminium hydride, sodium borohydride etc., aluminium

isopropylalcoholate, an alkaline metal or an alkaline metal amalgam as a catalyst in an alkanol as a hydrogen doner and as a solvent.

Aminoresorcinol derivatives of the general formula (IC) may be prepared from compounds of the general formula:

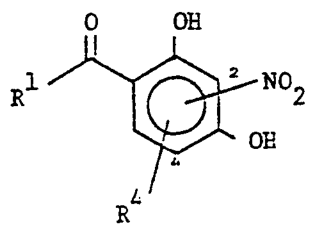

(III)

(wherein the various symbols are as hereinbefore defined and the nitro group and the group $R^4$ can be attached to the position 2 and 4, or 4 and 2 of the resorcinol ring, respectively) by selective reduction to convert the nitro group to a primary amine.

The reduction can be carried out, for example, by using hydrogen gas in the presence of a catalyst such as nickel, platinum or palladium-carbon, by using iron, zinc, tin or stannous chloride in addition to hydrochloric acid, by using titanium trichloride in the presence of benzene or a mixture of benzene and tetrahydrofuran, by using sodium sulfide or ammonium sulfide in water or an aqueous alcohol, or by using sodium hydrosulfite in ammonia water etc.

The compounds of the general formula (III) may be prepared from compounds of the general formula:

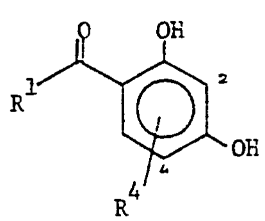

(IV)

(wherein the various symbols are as hereinbefore defined and the group $R^4$ can be attached to the position 2 or 4 of the resorcinol ring) by nitration.

The nitration is carried out by using a conventional nitrating reagent, for example, a mixture of nitric acid and sulfuric acid, a mixture of nitric acid and sulfuric acid in acetic acid, or nitric acid in acetic acid.

When the group $R^4$ is a hydrogen atom, the product of the general formula (III) is a mixture of isomers in which the nitro group is attached to the position 2 or 4 of the resorcinol ring. Each isomer may be separated by a conventional method of separation, e.g. by distillation under reduced pressure or by high-speed liquid, column or thin layer chromatography on silica gel, or by recrystallization.

The compounds of the general formula (IV) are well known *per se* (e.g. 6-benzoylresorcinol is a compound on the market) or may be prepared by methods known *per se*, for example, by the series of reactions depicted schematically below in Scheme B, wherein the various symbols are as hereinbefore defined and the group $R^4$ can be attached to the position 2 or 4 of the resorcinol ring.

Scheme B

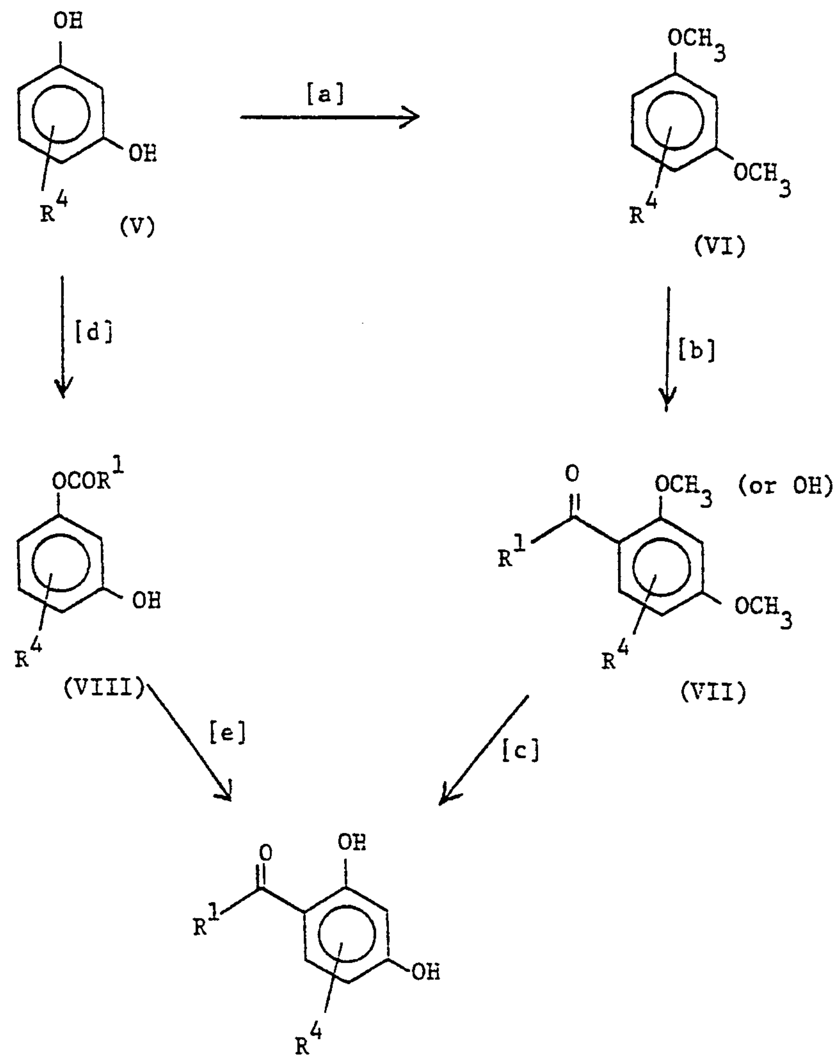


(V) (VI) (VIII) (VII)

(IV)

In Scheme B, each step can be effected using methods known *per se*. For example, step [a] may be carried out by etherification, by heating with methyl iodide in the presence of potassium carbonate in acetone.

Step [b] may be carried out by acylation of Friedel-Crafts reaction, for example, by reacting with an acylating agent such as acyl halides of the general formula: $R^1COX$ (in which X is a halogen atom and $R^1$ is as hereinbefore defined) in an inert organic solvent such as chloroform, methylene chloride, carbon tetrachloride in the presence of a catalyst such as aluminium chloride at a temperature from -20°C to 50°C.

Step [c] may be carried out by using hydrobromic acid or a mixture of hydrobromic acid and hydroiodic acid in acetic acid or without solvent, with refluxing.

Step [d] may be carried out by reacting with an acyl halide of the general formula: $R^1COX$ (in which the various symbols are as hereinbefore defined).

Step [e] may be carried out by Fries rearrangement, for example, in an inert organic solvent such as methylene chloride or without solvent, in the presence of a catalyst such as aluminium chloride.

Acid addition salts of aminoresorcinol derivatives of the general formula (I) may be prepared from the compounds of the general formula (I) by methods known *per se*, for example, by reacting stoichiometric quantities of a compound of the general formula (I) and an appropriate acid, e.g. an inorganic acid such as hydrochloric acid, hydrobromic acid, hydroiodic acid, sulfuric acid, phosphoric acid or nitric acid, or an organic acid such as acetic acid, lactic acid, tartric

acid, benzoic acid, citric acid, methanesulphonic acid, ethanesulphonic acid, benzenesulphonic acid, toluenesulphonic acid or isethionic acid, in a suitable solvent.

Preferably, acid addition salts are non-toxic salts. By the term 'non-toxic' as used in this specification, is meant salts the anions of which are relatively innocuous to animal organism when used in therapeutic doses so that the benefitical pharmacological properties of the compounds of general formula (I) are not vitiated by side effects ascribable to those anions. Preferably the salts are water-soluble. Suitable acid addition salts are, for example, inorganic acid salts such as hydrochloride, hydrobromide, hydroiodide, sulfate, phosphorate, nitrate, or organic acid salts such as acetate, lactate, tartrate, benzoate, citrate, methanesulphonate, ethanesulphonate, benzenesulphonate, toluenesulphonate or isethionate.

Aminoresorcinol derivatives of the general formula (I) and their pharmaceutically-acceptable acid addition salts possess an inhibitory effect on 5-lipoxygenase specifically, or 5-lipoxygenase and cyclooxygenase at the same time, and therefore, effective when it is desired to control the biosynthesis of leukotrienes and various PGs in mammals including human, especially human.

For example, in standard assay system using polymorphonuclear leukocytes from guinea pig (details of experiments were described hereafter), the concentrations of 2-amino-6-benzoylresorcinol hydrochloride for 50% inhibitions on 5-lipoxygenase and on cyclooxygenase were 0.3 μM and 0.8 μM, respectively. Therefore, it is confirmed that this compound inhibits both enzymes at the same time at a very low concentration.

The experiments for screening using polymorphonuclear leukocytes were carried out as follows: The reaction mixture (0.1 ml) containing 5 μmole of potassium phosphate at pH 7.4, 0.1 μmole of calcium chloride, polymorphonuclear leukocytes prepared from guinea pigs (4 x $10^7$ cells) and test compound, was preincubated for 5 minutes at 37°C. The reaction was started by the addition of [$^{14}C$] arachidonic acid (2.2 x $10^5$ dpm, 1.8 nmole), followed by the incubation at 37°C for 5 minutes. The reaction was terminated by the addition of 0.3 ml of a mixture of ether/methanol/1M citric acid (30/4/1). The organic phase (20 μl) was applied to silica gel plate. Thin layer chromatography was performed to separate each product with solvent system of ether/petroleum ether/acetic acid (85/15/0.1).

Activity of 5-lipoxygenase and cyclooxygenase were calculated from the measurements of the production of 5-HETE, and 12L-hydroxy-5,8,10-heptatrienoic acid (i.e. HHT) or thromboxane $B_2$ (i.e. $TXB_2$), respectively [it has been known that HHT and $TXB_2$ are the last products of the system concerning cyclooxygenase in the arachidonate cascade]. The concentrations for 50% inhibitions on 5-lipoxygenase and on cyclooxygenase were evaluated by using the concentration of compounds of the present invention required 50% inhibitions on the productions of 5-HETE, and HHT or $TXB_2$, respectively.

Further, in another assay system, the inhibitory activity of compounds of the present invention on 5-lipoxygenase only was also evaluated by using an enzyme prepared from polymorphonuclear leukocytes of guinea pig. Guinea pig was treated by casein and harvested polymorphonuclear leukocytes from cavity were sonicated, followed by

the centrifugation at 105,000 x g. The supernate was used as an enzyme throughout this work. The supernate (100 - 500 μg of protein) was incubated for 5 minutes at 30°C with [$^{14}C$] arachidonic acid and 1 mM calcium chloride in the presence or in the absence of test compound. The reaction was terminated by the addition of ether. Ether extract was separated by thin layer chromatography using silica gel plates to observe production of 5-HETE, 5,12-diHETE and LTB.

In this assay, the concentrations of 2-amino-6-benzoylresorcinol hydrochloride, 4-amino-6-benzoylresorcinol hydrochloride, 2-amino-4-chloro-6-(4-chlorobenzoyl)resorcinol hydrochloride and 2-amino-4-chloro-6-(α-methoxy-4-chlorobenzyl)resorcinol hydrochloride required to produce a 50% inhibition on the productions of 5-HETE, 5,12-diHETE and LTB (concentration for a 50% inhibition of 5-lipoxygenase, i.e. $IC_{50}$) were 0.4 μM, 6.67 μM, 2.63 μM and 2.0 μM, respectively.

On the other hand, all extent of the compounds of the present invention were confirmed that their acute toxicities were more than 30 mg/kg by intravenous administration. Therefore, aminoresorcinol derivatives of the present invention may be considered to be sufficiently safe and suitable for medical use. For Example, no mouse was dead when 2-amino-6-benzoylresorcinol hydrochloride was intravenously administered to tail vein in seven mice at the dose of 30 mg/kg.

To control the biosynthesis of leukotrienes and prostaglandins are useful for the prevention and the treatment of allergic tracheal and bronchial diseases or allergic lung diseases, allergic shocks or various allergic inflammations, in addition to various inflammations induced by prostaglandins in mammals including human , especially human. For such

purpose, the compounds of this invention are usually administered systemically, for example, orally, rectally or parenterally. Doses are determined depending upon age, symptoms, the desired therapeutic effects, the route of administration, the duration of the treatment and the like, and are generally and preferably about 0.1 mg to 500 mg for oral administration, and 1 μg to 1 mg for intravenous injection or 0.1 μg to 0.1 mg/hour for intravenous infusion, specially when required emergency treatment.

Solid compositions for oral administration include compressed tablets, pills, dispersible powders and granules. In such solid compositions, one or more of the active compounds is or are, admixed with at least one inert diluent such as calcium carbonate, potato starch, dextrin, alginic acid, lactose, mannitol, glucose and cacao butter. The compositions may also comprise, as is normal practice, additional substances other than inert diluents e.g. lubricating agents such as magnesium stearate or disintegrating agents such as cellulose calcium gluconate. The tablets or pill may, if desired, be coated and made into sugar-coated, gelatin-coated, enteric-coated or film-coated tablets and pills, or tablets or pills may be coated with two or more layers.

Liquid compositions for oral administration include pharmaceutically acceptable emulsions, solutions, suspensions, syrups and elixirs containing inert diluents commonly used in the art, such as water or liquid paraffin. Besides inert diluents such compositions may also comprise adjuvatns such as wetting and suspending agents, and sweetening, flavouring, perfuming and preservating agents.

The compositions according to this invention for oral

administration, also include capsules of absorbable materials such as gelatin containing one or more of the active substances with or without the addition of diluents or excipients.

Solid compositions for intrarectal administration include suppositories formulated in manner known *per se* and containin one or more of the active compounds.

Preparation according to this invetion for parenteral administration include sterile aqueous or non-aqueous solutions, suspensions or emulsions. Examplesof non-aqueous solvents or suspending media are propylene glycol, polyethylene glycol, ethanol, vegetable oils such as olive oil, and injectable organic esters such as ethyl oleate or sorbitan esters. These compositions may also include adjuvants such as preserving, wetting, emulsifying and dispersing agents. They may be sterilized, for example, by filtration through a bacteria-retaining filter, by incorporation of sterilizing agents in the compositions or by irradiation. They may also be manufactured in the form of sterile solid compositions which can be dissolved in sterile water or some other sterile injectable medium immediately before use.

The percentage of active ingredient in the compositions of the present invention may be varied, it being necessary that it should constitute a proportion such that a suitable dosage for the desired therapeutic effect shall be obtained. Several unit dosage forms may of course be administered at the same time. In general the preparations should normally contain at least 0.025% by weight of active substance when required for administration by injection: for oral administration the preparations will normally contain at least 0.1 wt % of active substance.

Preferred compounds of the present invention are, for example, as follows:

2-amino-6-hexanoylresorcinol,

2-amino-6-heptanoylresorcinol,

2-amino-6-benzoylresorcinol,

4-amino-6-benzoylresorcinol,

2-amino-6-(α-hydroxybenzyl)resorcinol,

4-amino-6-(α-hydroxybenzyl)resorcinol,

2-amino-6-(α-methoxybenzyl)resorcinol,

4-amino-6-(α-methoxybenzyl)resorcinol,

2-amino-6-(α-ethoxybenzyl)resorcinol,

4-amino-6-(α-ethoxybenzyl)resorcinol,

2-amino-6-(4-chlorobenzoyl)resorcinol,

2-amino-6-(α-hydroxy-4-chlorobenzyl)resorcinol,

2-amino-6-(α-methoxy-4-chlorobenzyl)resorcinol,

2-amino-6-(4-methylbenzoyl)resorcinol,

2-amino-6-(α-hydroxy-4-methylbenzyl)resorcinol,

2-amino-6-(α-methoxy-4-methylbenzyl)resorcinol,

2-amino-6-(4-methoxybenzoyl)resorcinol,

2-amino-6-(α-hydroxy-4-methoxybenzyl)resorcinol,

2-amino-6-(α-methoxy-4-methoxybenzyl)resorcinol,

2-amino-4-chloro-6-benzoylresorcinol,

2-amino-4-chloro-6-(α-hydroxybenzyl)resorcinol,

2-amino-4-chloro-6-(α-methoxybenzyl)resorcinol,

2-amino-4-chloro-6-(4-chlorobenzoyl)resorcinol,

2-amino-4-chloro-6-(α-hydroxy-4-chlorobenzyl)resorcinol,

2-amino-4-chloro-6-(α-methoxy-4-chlorobenzyl)resorcinol,

2-amino-4-chloro-6-(4-methylbenzoyl)resorcinol,

2-amino-4-chloro-6-(α-hydroxy-4-methylbenzyl)resorcinol,

2-amino-4-chloro-6-(α-methoxy-4-methylbenzyl)resorcinol,

2-amino-4-chloro-6-(4-methoxybenzoyl)resorcinol,

2-amino-4-chloro-6-(α-hydroxy-4-methoxybenzyl)resorcinol,

2-amino-4-chloro-6-(α-methoxy-4-methoxybenzyl)resorcinol,

2-amino-4-fluoro-6-benzoylresorcinol,

2-amino-4-fluoro-6-(α-hydroxybenzyl)resorcinol,

2-amino-4-fluoro-6-(α-methoxybenzyl)resorcinol,

2-amino-4-fluoro-6-(4-chlorobenzoyl)resorcinol,

2-amino-4-fluoro-6-(α-hydroxy-4-chlorobenzyl)resorcinol,

2-amino-4-fluoro-6-(α-methoxy-4-chlorobenzyl)resorcinol,

2-amino-4-fluoro-6-(4-methylbenzoyl)resorcinol,

2-amino-4-fluoro-6-(α-hydroxy-4-methylbenzyl)resorcinol,

2-amino-4-fluoro-6-(α-methoxy-4-methylbenzyl)resorcinol,

2-amino-4-fluoro-6-(4-methoxybenzoyl)resorcinol,

2-amino-4-fluoro-6-(α-hydroxy-4-methoxybenzyl)resorcinol,

2-amino-4-fluoro-6-(α-methoxy-4-methoxybenzyl)resorcinol,

2-amino-4-tert-butyl-6-benzoylresorcinol,

2-amino-4-tert-butyl-6-(α-hydroxybenzyl)resorcinol,

2-amino-4-tert-butyl-6-(α-methoxybenzyl)resorcinol,

2-amino-4-tert-butyl-6-(4-chlorobenzoyl)resorcinol,

2-amino-4-tert-butyl-6-(α-hydroxy-4-chlorobenzyl)resorcinol,

2-amino-4-tert-butyl-6-(α-methoxy-4-chlorobenzyl)resorcinol,

2-amino-4-tert-butyl-6-(4-methylbenzoyl)resorcinol,

2-amino-4-tert-butyl-6-(α-hydroxy-4-methylbenzyl)resorcinol,

2-amino-4-tert-butyl-6-(α-methoxy-4-methylbenzyl)resorcinol,

2-amino-4-tert-butyl-6-(4-methoxybenzoyl)resorcinol,

2-amino-4-tert-butyl-6-(α-hydroxy-4-methoxybenzyl)resorcinol,

2-amino-4-tert-butyl-6-(α-methoxy-4-methoxybenzyl)resorcinol,

and acid addition salts thereof.

The following Reference Examples and Examples illustrate, but not limit, the preparation of compounds of the present invention. In the Reference Examples and Examples, 'TLC', 'IR', 'NMR' and 'Mass' represent 'Thin layer chromatography', 'Infrared absorption spectrum', 'Nuclear magnetic resonance' and 'Mass spectrum', respectively. Where solvent ratios are specified in chromatographic separations, the ratios are by volume. The solvents in parentheses in 'TLC' show the developing solvent used. Except when specified otherwise, 'IR' is recorded by KBr tablet method and 'NMR' is recorded in deuterochloroform ($CDCl_3$) solution.

Reference Example 1

4-Chloro-O,O'-dimethylresorcinol

Under an atmosphere of nitrogen, to a solution of 25 g of 4-chlororesorcinol (being on the market) in 500 ml of acetone was added 50 ml of methyl iodide and 50 g of potassium carbonate at room temperature and the mixture was refluxed for 5 hours. The reaction mixture was concentrated under reduced pressure and to the residue thus obtained was added cyclohexane. Insoluble salts were filtered off and the filtrate was washed with water, dried and concentrated under reduced pressure to give 30.381 g of the crude title compound having the following physical characteristics:

IR(neat): $\nu$=1600, 1585, 1490, 1310, 1210, 1160, 1075, 1030 $cm^{-1}$;

NMR: $\delta$=7.25 (1H, d), 6.6-6.3 (2H, m), 3.87 (3H, s), 3.78 (3H, s);

Mass: m/e=172 ($M^+$).

Reference Example 2

4-Chloro-6-(4-chlorobenzoyl)-3-O-methylresorcinol

Under an atmosphere of nitrogen, to a suspension of 21 g of aluminium chloride in 100 ml of methylene chloride was added dropwise 25 g of 4-chlorobenzoyl chloride (being on the market) at 0°C, and then to the mixture was added dropwise 24.668 g of 4-chloro-O,O'-dimethyl-resorcinol (prepared in Reference Example 1) in 50 ml of methylene chloride at room temperature. After stirring for 20 hours at the same temperature, the reaction mixture was poured into a mixture of ice water and hydrochloric acid and extracted with methylene chloride. The extract was washed with a saturated aqueous solution of sodium bicarbonate, dried and concentrated under reduced pressure to give crude crystals. The crystals thus obtained were washed with a mixture of cyclohexane and ethyl acetate (10:1) and dried to give 28 g of the title compound having the following physical characteristics:

TLC (benzene): Rf=0.55;

IR: ν=1620, 1585, 1380, 1345, 1265, 1210, 1090, 1055, 1010, 920, 840, 810, 770, 710 $cm^{-1}$;

NMR: δ=12.48 (1H, s), 7.7-7.4 (5H, m), 6.59 (1H, s), 3.97 (3H, s);

Mass: m/e=296 ($M^+$), 185.

Reference Example 3

4-Chloro-6-(4-chlorobenzoyl)resorcinol

Under an atmosphere of nitrogen, a mixture of 3 g of 4-chloro-6-(4-chlorobenzoyl)-3-O-methylresorcinol (prepared in Reference Example 2), 200 ml of acetic acid and 20 ml of a 47% aqueous solution of

hydrobromic acid was refluxed for 18 hours. After cooling, diethyl ether was added to the reaction mixture and further ice water was added thereto to precipitate unreacted starting materials. The precipitate was filtered off and the filtrate was extracted with a mixture of cyclohexane and ethyl acetate (10:1) and the extract was washed with water and a saturated aqueous solution of sodium bicarbonate successively, dried and concentrated under resuced pressure. The precipitated crystals thus obtained were washed with cyclohexane and dried to give 642 mg of the title compound having the following physical characteristics:

Melting point: 170° - 190°C;

TLC (cyclohexane:ethyl acetate=2:1): Rf=0.36;

IR: ν=3250, 1620, 1600, 1340, 1260, 1225, 920 $cm^{-1}$;

NMR: δ=7.85-7.4 (4H, m), 7.48 (1H, s), 6.66 (1H, s);

Mass: m/e=282 ($M^+$), 247, 171.

Reference Example 4

6-Benzoyl-4-nitroresorcinol and 6-benzoyl-2-nitroresorcinol

To a solution of 2.14 g of 6-benzoylresorcinol (being on the market) in 10 ml of acetic acid, 1 g of concentrated nitric acid was added dropwise over a period of an hour at room temperature, and the solution was stirred at room temperature for an hour to precipitate crystals. After sirring the reaction mixture with water, the crystals were separated and dried under reduced pressure to obtain 1.9 g of the crude title compounds. The crude compounds were separated by column chromatography on silica gel using a mixture of methylene chloride and methanol (50:1) as an eluent to give the title compounds having the

following physical characteristics:

(a) 6-Benzoyl-4-nitroresorcinol

Yield: 1.0 g;

TLC (methylene chloride:methanol=10:1): Rf=0.86;

IR: ν=3450, 3280, 3100, 3060, 1635, 1605, 1570, 1530, 1480, 1440, 1355, 1325, 1310, 1295, 1160, 1115, 1080, 930, 910, 855, 820, 800, 765, 740, 700 $cm^{-1}$;

NMR (deuterochloroform + methanol-$d_4$ + acetone-$d_6$ solution): δ=8.37 (1H, s), 7.51 (5H, s), 6.58 (1H, s);

Mass: m/e=259 ($M^+$), 258, 212, 182, 136, 105, 77.

(b) 6-Benzoyl-2-nitroresorcinol

Yield: 145 mg;

TLC(methylene chloride:methanol=10:1): Rf=0.42;

IR: ν= 3450, 1605 (broad), 1525, 1485, 1440, 1330, 1285, 1255, 1200, 1145, 1060, 980, 805, 780, 740, 700 $cm^{-1}$;

NMR: δ=11.23 (1H,s), 7.61 (1H, d), 7.44 (5H, s), 6.51 (1H, d);

Mass: m/e=259($M^+$), 258, 213, 182, 164, 105, 77.

Using the same procedure, the compound having the following physical characteristics was obtained.

(c) 4-Chloro-6-(4-chlorobenzoyl)-2-nitroresorcinol

Starting material: 283 mg of 4-chloro-6-(4-chlorobenzoyl)resorcinol (prepared in Reference Example 3);

Yield: 165 mg;

Melting point: 120°C;

TLC (ethyl acetate): Rf=0.25;

IR: ν=1620, 1595, 1535, 1330, 1260, 1180, 1085, 985 $cm^{-1}$;

NMR: δ=7.88 (1H, s), 7.75-7.4 (4H, m);

Mass: m/e=327 ($M^+$).

Example 1

4-Amino-6-benzoylresorcinol hydrochloride

To a solution of 400 mg of 6-benzoyl-4-nitroresorcinol (prepared in Reference Example 4(a)) in 5 ml of chloroform, 20 ml of ethanol was added, and further, 100 mg of palladium-carbon (content: 10%) was also added to the solution. The atmosphere in the reaction vessel was replaced with hydrogen gas and the mixture was stirred at room temperature for two hours. Catalyst was removed off by filtration and the filtrate was concentrated under reduced pressure. The residue was dissolved in ethanol and thereto 0.2 ml of concentrated hydrochloric acid was added. The solution was concentrated under reduced pressure and dried by vacuum pump. The residue thus obtained was dissolved in one ml of methanol, and 50 ml of diethyl ether was added to the solution in order to reprecipitating, to give 350 mg of the title compound having the following physical characteristics:

Melting point: 155°-160°C (decomposed);

TLC (methylene chloride:methanol=10:1): Rf=0.42;

IR: ν=3450, 3050, 2900 (broad), 2580, 1640, 1620 (shoulder), 1600, 1570, 1500, 1445, 1380, 1345, 1280, 1175, 1115, 920, 845, 740, 700 $cm^{-1}$;

NMR (methanol-$d_4$ solution): δ=7.6 (6H, m), 6.62 (1H, s);

Mass: m/e=229 ($M^+$), 228, 152, 151, 123, 105.

Using the same procedure, the compound having the following physical characteristics was obtained.

(a) 2-Amino-6-benzoylresorcinol hydrochloride

Starting material: 145 mg of 6-benzoyl-2-nitroresorcinol (prepared in Reference Example 4(b));

Yield: 91 mg;

Melting point: 175° - 185°C (decomposed);

TLC (methylene chloride : methanol = 10 : 1): Rf = 0.45;

IR: ν =3450, 3050, 2920, 2790, 2590, 1640, 1620 (shoulder), 1570, 1510, 1445, 1305, 1295 (shoulder), 1190, 1105, 1050, 1025, 950, 800, 765, 695 $cm^{-1}$;

NMR (methanol-$d_4$ solution): δ=7.61 (5H, m), 7.59 (1H, d), 6.63 (1H, d);

Mass: m/e=229 ($M^+$), 152, 151, 123, 105.

Example 2

2-Amino-4-chloro-6-(4-chlorobenzoyl)resorcinol hydrochloride

To a solution of 50 mg of 4-chloro-6-(4-chlorobenzoyl)-2-nitroresorcinol (prepared in Reference Example 4(c)) in one ml of water, was added 0.5 ml of concentrated aqueous ammonia, a solution of 350 mg of sodium hydrosulfide in 1.5 ml of water and further tetrahydrofuran in order to dissolve the solid at room temperature, and the mixture was stirred for 15 minutes at the same temperature. The reaction mixture was diluted with ethyl acetate, washed with a saturated aqueous solution of sodium chloride, dried and concentrated under reduced pressure.
The precipitated crystals thus obtained was dissolved in a proper amount of tetrahydrofuran, and 0.5 ml of hydrochloric acid was added thereto, and the solution was concentrated under reduced pressure. The precipitated crystals thus obtained were washed with a mixture of

chloroform and cyclohexane (1:1) and dried by vacuum pump to give 26 mg of the title compound having the following physical characteristics:

Melting point: 180°C;

TLC (methylene chloride:methanol=10:1): Rf=0.48;

IR: ν=1630, 1595, 1480, 1430, 1300, 1090, 780 $cm^{-1}$;

NMR (methanol-$d_4$ solution): δ=7.70 (2H, d), 7.60 (2H, d), 7.49 (1H, s);

Mass: m/e=297 ($M^+$), 187, 185, 111.

Example 3

2-Amino-4-chloro-6-(α-methoxy-4-chlorobenzyl)resorcinol hydrochloride

Under an atmosphene of nitrogen, to a solution of 100 mg of 2-amino-4-chloro-6-(4-chlorobenzoyl)resorcinol hydrochloride (prepared in Example 2) in 5 ml of methanol was added 114 mg of sodium borohydride at 0°C and the mixture was stirred gently. The reaction mixture was neutralized with acetic acid and concentrated under reduced pressure. The residue thus obtained was extracted with ethyl acetate and the extract was washed with a saturated aqueous solution of sodium chloride, dried and concentrated under reduced pressure to give 2-amino-4-chloro-6-(α-hydroxy-4-chlorobenzyl)resorcinol. The product thus obtained was dissolved in a proper amount of methanol and 0.2 ml of hydrochloric acid was added thereto and the mixture was concentrated under reduced pressure. To the obtained residue was added a proper amount of chloroform and the solution was concentrated under reduced pressure. The precipitated crystals obtained were washed with a mixture of chloroform and cyclohexane (1:1) to give 56 mg of the title compound having the following physical characteristics:

Melting point: 170°-180°C (decomposed);

TLC (chloroform:methanol=10:1): Rf=0.65;

IR: ν=1485, 1300, 1100, 860 $cm^{-1}$;

NMR (methanol-$d_4$ solution): δ=7.46-7.3 (4H, m), 7.12 (1H, s), 5.61 (1H, s), 3.43 (3H, s);

Mass: m/e=313 ($M^+$), 281, 246.

Example 4

5 g of 2-amino-6-benzoylresorcinol hydrochloride, 200 mg of cellulose magnesium gluconate (disintegrator), 100 mg of magnesium stearate (lubricating agent) and 4.7 g of crystal cellulose were mixed and punched out in a conventional method to obtain 100 tablets containing 50 mg of the active ingredient in one tablet.

Claims:

1. An aminoresorcinol derivative of the general formula:

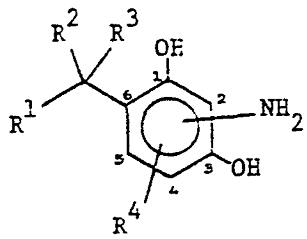

(I)

[wherein $R^1$ represents an alkyl group containing from 1 to 6 carbon atoms, or a group of the general formula:

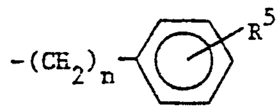

(II)

(in which $R^5$ represents a hydrogen atom, a halogen atom, an alkyl group containing from 1 to 4 carbon atoms, an alkoxy group containing from 1 to 4 carbon atoms, a carboxy group, an amino group, a carbamoyl group, a sulfamoyl group or a cyano group and n represents zero or an integer of 1 or 2), one of $R^2$ and $R^3$ represents a hydrogen atom and the other represents a hydroxy group or an alkoxy group containing from 1 to 4 carbon atoms or $R^2$ and $R^3$ together represent an oxo group, and $R^4$ represents a hydrogen atom, a halogen atom, an alkyl group containing from 1 to 6 carbon atoms or a phenylthio group, and the amino group and the group $R^4$ in the general formula (I) can be attached to the position 2 and 4, or 4 and 2 of the resorcinol ring, respectively], or a pharmaceutically-acceptable acid addition salt thereof.

2. A compound according to claim 1 wherein $R^1$ represents a group of the general formula:

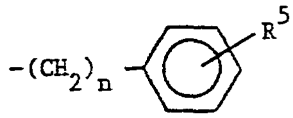

(II)

(wherein the various symbols are as defined in claim 1).

3. A compound according to claim 2, wherein $R^5$ represents a hydrogen atom, a halogen atom, an alkyl group containing from 1 to 4 carbon atoms or an alkoxy group containing from 1 to 4 carbon atoms.

4. A compound according to claim 3, wherein $R^5$ represents a hydrogen, chlorine or fluorine atom.

5. A compound according to claim 1, wherein $R^2$ and $R^3$ together represent an oxo group.

6. A compound according to claim 1, wherein one of $R^2$ and $R^3$ represents a hydrogen atom and the other represents a hydroxy, methoxy or ethoxy group.

7. A compound according to claim 1, wherein $R^4$ represents a hydrogen or halogen atom, or a tert-butyl group.

8. A compound according to claim 7, wherein $R^4$ represents a hydrogen, chlorine or fluorine atom.

9. A compound according to claim 1, which is 4-amino-6-benzoylresorcinol or its hydrochloride.

10. A compound according to claim 1, which is 2-amino-6-benzoylresorcinol or its hydrochloride.

11. A compound according to claim 1, which is 2-amino-4-chloro-6-(4-chlorobenzoyl)resorcinol or its hydrochloride.

12. A compound according to claim 1, which is 2-amino-4-chloro-6-(α-hydroxy-4-chlorobenzyl)resorcinol or its hydrochloride.

13. A compound according to claim 1, which is 2-amino-4-chloro-6-(α-methoxy-4-chlorobenzyl)resorcinol or its hydrochloride.

14. A process for the preparation of aminoresorcinol derivatives as claimed in claim 1 and conforming to the general formula:

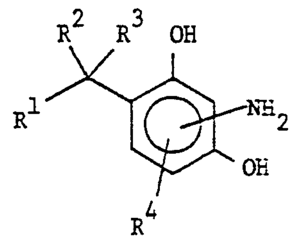

(I)

(wherein the various symbols are as defined in claim 1), or a pharmaceutically-acceptable acid addition salt thereof, which comprises (i) selectively reducing the nitro group of a compound of the general formula:

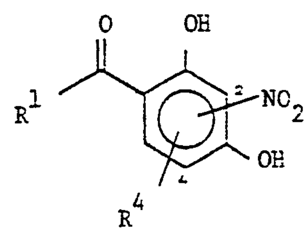

(III)

(wherein the various symbols are as hereinbefore defined and the nitro group and the group $R^4$ can be attached to the position 2 and 4, or 4 and 2 of the resorcinol ring, respectively) to convert to a primary amine to obtain a compound of the general formula:

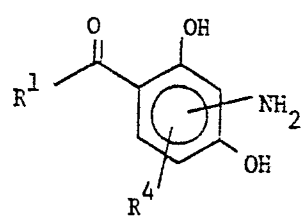

(IC)

(wherein the various symbols are as hereinbefore defined),

(ii) reducing the oxo group of a compound of the general formula:

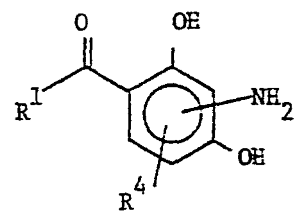

(IC)

(wherein the various symbols are as hereinbefore defined) to convert to a hydroxy group to obtain a compound of the general formula:

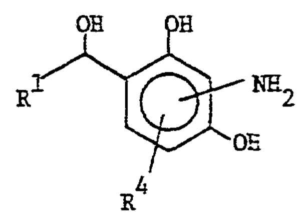

(IB)

(wherein the various symbols are as hereinbefore defined),

(iii) etherifying the secondary alcohol of a compound of the general formula:

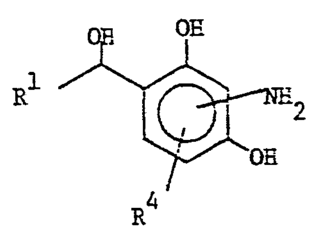

(IB)

(wherein the various symbols are as hereinbefore defined) to convert an alkoxy group to obtain a compound of the general formula:

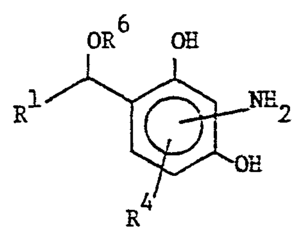

(IA)

(wherein $R^6$ represents an alkyl group containing from 1 to 4 carbon

atoms and the other symbols are as hereinbefore defined), or (iv) converting the aminoresorcinol derivative of the general formula (I) obtained into a pharmaceutically-acceptable acid addition salt thereof by methods known _per se_.

15. A pharmaceutical composition for the prevention and the treatment of allergic tracheal and bronchial diseases or allergic lung diseases, allergic shocks or various allergic inflammations, or for the prevention and the treatment of various inflammations induced by prostaglandins, which comprises, as active ingredient, an effective amount of at least one compound of the general formula (I) depicted in claim 1, wherein the various symbols are as defined in claim 1, or a pharmaceutically-acceptable acid addition salt thereof, together with a pharmaceutical carrier or coating.

16. A method for the prevention and treatment of allergic tracheal and bronchial diseases or allergic lung diseases, allergic shocks or various allergic inflammations, or for the prevention and treatment of various inflammations induced by prostaglandins, which comprises the systemical administration of an effective amount of at least one compound of the general formula (I) depicted in claim 1, wherein the various symbols are as defined in claim 1, or a pharmaceutically-acceptable acid addition salt thereof.

European Patent Office

**PARTIAL EUROPEAN SEARCH REPORT**
which under Rule 45 of the European Patent Convention shall be considered, for the purposes of subsequent proceedings, as the European search report

0079141

Application number
EP 82 30 5512.4

## DOCUMENTS CONSIDERED TO BE RELEVANT

| Category | Citation of document with indication, where appropriate, of relevant passages | Relevant to claim | CLASSIFICATION OF THE APPLICATION (Int Cl) |
|---|---|---|---|
| A | Chemical Abstracts vol. 86, no.21, 1977, 23 May 1977<br>Columbus, Ohio, USA<br>D. KOLBAH et al. "Stereoselective in-vitro aromatic-ring oxygenations of chiral 1,4-benzodiazepin-2-ones"<br>page 471, column 1, abstract no. 155622d<br>& Helv. Chim. Acta, vol. 60. no. 1, 1977 pages 265 to 283<br>Chemical Abstracts, Chemical Substance Index, I-Po, vol. 86, page 3142 CS<br>-- | 1-5,7, 8 | C 07 C 97/10<br>C 07 C 93/14<br>A 61 K 31/135 |
| | | | TECHNICAL FIELDS SEARCHED (Int Cl) |
| A | US - A - 3 428 644 (SANDOZ INC.)<br>* column 1; claim 1 *<br>-- | 1-5,7, 8 | C 07 C 93/00<br>C 07 C 97/00 |
| A | US - A - 2 763 657 (EASTMAN KODAK)<br>* example 1 *<br>--<br>./.. | 1-5,7, 8 | |

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 1-15
Claims searched incompletely: -
Claims not searched: 16
Reason for the limitation of the search:

method for treatment of the human body, article 52 (4)

CATEGORY OF CITED DOCUMENTS

X: particularly relevant if taken alone
Y: particularly relevant if combined with another document of the same category
A: technological background
O: non-written disclosure
P: intermediate document
T: theory or principle underlying the invention
E: earlier patent document, but published on, or after the filing date
D: document cited in the application
L: document cited for other reasons

&: member of the same patent family, corresponding document

| Place of search | Date of completion of the search | Examiner |
|---|---|---|
| Berlin | 12-01-1983 | BREW |

EPO Form 1505.1 06.78

0079141

European Patent Office

# PARTIAL EUROPEAN SEARCH REPORT

Application number EP 82 30 5512.4



| DOCUMENTS CONSIDERED TO BE RELEVANT | | | CLASSIFICATION OF THE APPLICATION (Int. Cl.3) |
|---|---|---|---|
| Category | Citation of document with indication, where appropriate, of relevant passages | Relevant to claim | |
| A | GB - A - 809 791 (N.V. PHILIPS' GLOEILAMPENFABRIEKEN)<br>* claim 3; page 1, lines 23 to 25 *<br>---- | 1-5,7, 8,15 | |
| | | | TECHNICAL FIELDS SEARCHED (Int. Cl.3) |